# EUROPÄISCHE PATENTSCHRIFT

(11) **EP 0 880 967 B1**
(45) Veröffentlichungstag und Bekanntmachung des Hinweises auf die Patenterteilung: **04.09.2002**
(21) Anmeldenummer: 98109336.2
(22) Anmeldetag: 22.05.1998
(51) Int. Cl.: A61K 31/505

(54) **Nephroprotektive Arzneimittel**
Nephroprotective drug
Médicament néphroprotecteur

(30) Priorität: 28.05.1997 DE 19722322
(43) Veröffentlichungstag der Anmeldung: 02.12.1998
(73) Patentinhaber: Solvay Pharmaceuticals GmbH, 30173 Hannover (DE)
(72) Erfinder: Ritz, Eberhard, Prof. Dr., 69126 Heidelberg (DE); Ammann, Kerstin, Dr., 69120 Heidelberg (DE); Bielenberg, Gerhard Wilhelm, Dr., 31061 Alfeld/Leine (DE)
(74) Vertreter: Gosmann, Martin

(56) Entgegenhaltungen:
- WO-A-96/26728
- HOHAGE ET AL.: "Effects of moxonidine and clonidine on renal function and blood pressure in anesthetized rats" CLINICAL NEPHROLOGY, Bd. 47, Nr. 5, Mai 1997, Seiten 316-324, XP002087574
- TÖRNIG ET AL.: "Arteriolar wall thickening, capillary rarefaction and interstitial fibrosis in the heart..." JOURNAL OF THE AMERICAN SOCIETY OF NEPHROLOGY, Bd. 7, Nr. 5, Mai 1996, Seiten 667-675, XP002087575
- WIECEK ET AL.: "Effect of moxonidine on urinary electrolyte excretion and renal haemodynamics in man" EUROPEAN JOURNAL CLINICAL PHARMACOLOGY, Bd. 48, Nr. 3/4, 1995, Seiten 203-208, XP002087576
- GERHARD MALL ET. AL: 'Modern antihypertensives and nephroprotection' CARDIOVASCULAR RISK FACTORS Bd. 5, Nr. 1, Seiten 33 - 39

## Beschreibung

Die vorliegende Erfindung betrifft die Verwendung von 4-Chlor-5-[(4,5-dihydro-1H-imidazol-2-yl)-amino]-6-methoxy-2-methylpyrimidin (= Moxonidin) und dessen physiologisch verträglichen Säureadditionssalzen zur Behandlung und/oder Prophylaxe von Niereninsuffizienz in normotensiven Patienten, und zur Herstellung von für diese Behandlung geeigneten Arzneimitteln.

Der Erfindung liegt die Aufgabe zugrunde, neue pharmazeutische Zubereitungen zur Behandlung von Niereninsuffizienz in normotensiven Patienten zu entwickeln.

Erfindungsgemäß werden zur Herstellung von pharmazeutischen Zubereitungen zur blutdrucksenkungsunabhängigen Behandlung von Niereninsuffizienz, in normotensiven Patienten, 4-Chlor-5-[(4,5-dihydro-1H-imidazol-2-yl)-amino]-6-methoxy-2-methylpyrimidin der Formel I 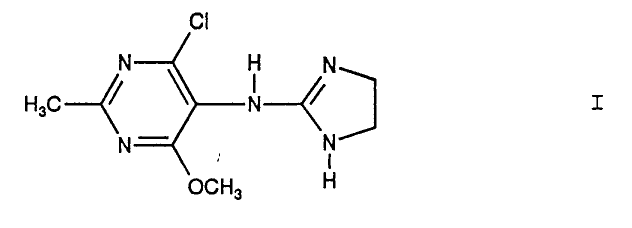 und dessen physiologisch verträgliche Säureadditionssalze in subantihypertensiven Mengen verwendet.

Als physiologisch verträgliche Säureadditionssalze des Moxonidins eignen sich Salze mit anorganischen Säuren, beispielsweise Halogenwasserstoffsäuren, oder mit organischen Säuren, beispielsweise niederen aliphatischen Mono- oder Dicarbonsäuren wie Essigsäure, Fumarsäure oder Weinsäure oder aromatischen Carbonsäuren wie z. B. Salicylsäure.

Die erfindungsgemäß zur blutdrucksenkungsunabhängigen Behandlung von Niereninsuffizienz eingesetzten Verbindungen fallen unter den Umfang von in der deutschen Offenlegungsschrift Nr. 28 49 537 beschriebenen 5-[(2-Imidazolin-2-yl)-amino]-pyrimidin-Derivaten mit blutdrucksenkenden Eigenschaften, und sind aus dieser Patentanmeldung bekannt. Moxonidin-haltige pharmazeutische Zubereitungen sind als Antihypertensiva unter dem Markennamen Physiotens^{R} im Handel erhältlich und werden medizinisch als Antihypertensivum eingesetzt. Die Verbindungen können auf an sich bekannte Weise nach den in der vorgenannten DOS beschriebenen Verfahren oder analog zu diesen Verfahren hergestellt werden.

Es ist bekannt, daß zwischen Bluthochdruck und chronischer Niereninsuffizienz Wechselbeziehungen bestehen, indem einerseits Bluthochdruck die Nieren stark belastet und Schädigungen der Nierenfunktion zur Folge haben kann und andererseits chronisch verminderte Nierenfunktion oft Bluthochdruck zur Folge hat. Aus einer Studie von Mall et al. (Cardiovascular Risk Factors Vol 5, Suppl 1, 1995, Seiten 33-39) ist ferner bekannt, daß Blutdrucksenkung (z.B. durch Application von blutdrucksenkenden pharmazeutischen Wirkstoffen, u.a. Moxonidin) einen nephroprotektiven Einfluß hat und Nierenschädigungen mindern oder verhindern kann.

Es wurde nun überraschenderweise gefunden, daß Moxonidin und seine physiologisch verträglichen Säureadditionssalze bereits in subantihypertensiven Dosen eine nephroprotektive Wirkung am Menschen und größeren Säugetieren besitzen und zur Verlangsamung oder Hemmung der Progression von verminderter Nierenleistung führen. Somit eignet sich Moxonidin auch zur blutdrucksenkungsunabhängigen Prophylaxe und Behandlung von Nierenschädigungen und Niereninsuffizienz, z.B. in normotensiven Patienten. Als subantihypertensive Dosen werden im Rahmen der vorliegenden Erfindung Dosen bezeichnet, welche keine physiologisch relevante Blutdrucksenkung hervorrufen. Als normotensiv werden im Rahmen der vorliegenden Erfindung Patienten bezeichnet, welche entweder spontan normotensiv sind oder durch Einnahme von anderen blutdrucksenkenden Mitteln normotensiv gestellt wurden. Erfindungsgemäß wird eine im wesentlichen blutdrucksenkungsfreie Behandlung mit Moxonidin (d.h. eine Behandlung mit Dosen, die keine physiologisch oder pharmakologisch signifikante Blutdrucksenkung hervorrufen) zur Verhinderung oder Linderung von Nierenkrankheiten eingesetzt und kann eine Renormalisierung von geschädigter Nierenarchitektur fördern.

Zur erfindungsgemäßen Behandlung von Niereninsuffizienz können Moxonidin und seine physiologisch verträglichen Säureadditionssalze in üblichen pharmazeutischen Zubereitungen oral, intravenös oder auch transdermal verabreicht werden.

So können subantihypertensive nephroprotektiv wirksame Mengen der Verbindungen erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese festen Präparate können pharmazeutisch übliche anorganische und/oder organische Trägerstoffe wie z. B. Milchzucker, Talkum oder Stärke neben pharmazeutisch üblichen Hilfsmitteln, beispielsweise Gleitmitteln oder Tablettensprengmitteln, enthalten. Flüssige Präparate wie Lösungen, Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfsund/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die nephroprotektive Wirkung von Moxonidin im subantihypertensiven Dosisbereich wurde in Standard-Tierversuchen an Ratten nachgewiesen.

Als subantihypertensive Dosen werden erfindungsgemäß Dosen eingesetzt in einem Dosisbereich , in welchem bei der behandelten Spezies keine pharmakologisch relevanten blutdrucksenkenden Wirkungen auftreten. Zur Untersuchung des Einflusses von subantihypertensiven Dosen von Moxonidin auf die Progression von Niereninsuffizienz wurde das als Versuchsmodell für Nierenschädigungen bekannte Tiermodell der subtotal nephrektomierten Ratte eingesetzt. Hierbei wurde als allgemein akzeptierter Ersatzindikator (=surrogate marker) für den Grad der eingetretenen Nierenschädigung bzw. Verminderung der Nierenleistung der Glomerulosklerose-Index bestimmt.

### Versuchsbeschreibung

Männliche Sprague-Dawley Ratten wurden mit einer Diät gefüttert, die 40 g Protein und 0,6 g NaCl je 100 g enthielt. Nach einer Adaptationsphase von 7 Tagen wurde eine subtotale Nephrektomie vorgenommen. Dabei wurde in einer ersten Operation in Kurzzeitnarkose (Ketamin/Xylazin i.m.) durch Abklemmen und Ligieren der Nierenarterie und Verschieben der Nierenkapsel zur Erhaltung der Nebenniere eine Nephrektomie rechts durchgeführt. Nach 5 Tagen, zu einem Zeitpunkt, an dem eine kompensatorische Hypertrophie der linken Niere eingetreten ist, wurde in einer zweiten Operation in Narkose (Ketamin/Xylazin i.m.) eine Resektion des oberen und unteren Pols der linken Niere durchgeführt. Ab dem 3. Tag nach der zweiten Operation wurde einer Gruppe von 10 Tieren über den gesamten Untersuchungszeitraum von 12 Wochen Moxonidin in einer Tagesdosis von ca. 1,5 mg/kg über die Futterpellets p.o. appliziert. Eine Kontrolltiergruppe erhielt die gleiche Diät ohne Moxonidin, wobei über ein Paarfütterungsprotokoll sichergestellt wurde, daß behandelte und unbehandelte Tiere die gleiche Futtermenge zu sich nahmen. Bei einem Teil der Versuchstiere (4 Tiere je Versuchsgruppe) wurde eine permanente Kontrolle des Blutdrucks durchgeführt. Die Erfassung des Blutdruck-Parameters erfolgte dabei mit Hilfe telemetrischer Datenübertragung (Data Sciences International, St.Paul, MN, USA). Dazu wurde den Ratten ein mit einem Sender verbundener Druckkatheter in die Bauchaorta implantiert.

Bei Versuchsende wurden in Narkose (Ketamin/Xylazin i.m.) die Nieren nach Perfusionsfixation nach quantitativen stereologischen Methoden aufgearbeitet. Dazu wurde ein Katheter in die Bauchaorta eingeführt. Das vaskuläre System wurde über diesen Katheter nach Auswaschen mit einer Dextran-Lösung (Rheomakrodex; Schiwa Co., Glandorf) für 12 Minuten mit einem 0,2 molaren Phosphatpuffer, der 3 % Glutaraldehyd enthielt, bei einem Perfusionsdruck von 110 mmHg fixiert. Die Nieren wurden gewogen und in 1 mm dicke Scheiben geschnitten. Die Scheiben wurden in Paraffin eingebettet, es wurden 4 µm dicke Schnitte angefertigt und diese mit Hämatoxilin/Eosin angefärbt. Der Glomeruloskleroseindex wurde lichtmikrosko-pisch nach der Methode von Raij et al. (Raij L, Azar S, Keane W. Kidney Int. 1984; 26: 137-143) bestimmt. Dabei wurde in mindestens 1000 Glomeruli die Sklerose entsprechend der betroffenen Flächen in Schweregrade von 0 bis 4 eingeteilt (0= keine Schädigung; 1= bis 25%; 2 = 25 bis 50%; 3 = 50 bis 75%; 4 = 75 bis 100%).

Die Versuchsergebnisse sind in der nachfolgenden Tabelle wiedergegeben. Die in der Tabelle angegebenen Werte für den Glomerulo-sklerose-Index sind Mittelwerte +/- Standardabweichung der Messung an jeweils 10 Tieren. Die Blutdruckwerte sind Mittelwerte +/- Standardabweichung der telemetrisch erfaßten Blutdruckmessung an jeweils 4 Tieren. Die Druckwerte sind als mittlerer systolischer Blutdruck über jeweils 24 Stunden in der 1. bzw. 9. Behandlungswoche angegeben.

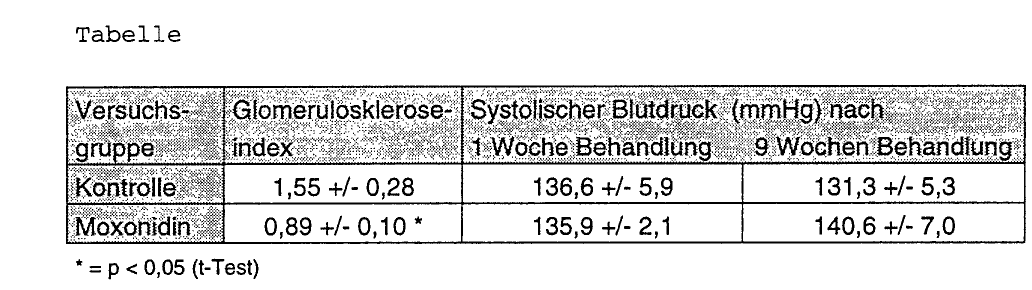

Die vorstehenden Versuchsergebnisse zeigen, daß unter Moxonidinbehandlung bei etwa gleichbleibenden Blutdruckwerten der Glomerulosklerose-Index um über 40% verringert ist. Dies ist ein Indiz für die Hemmwirkung des Moxonidins auf die Progression von Verminderung der Nierenleistung und von Nierenschädigungen.

Deshalb sind Moxonidin und seine Säureadditionssalze zur blutdrucksenkungsunabhängigen Prophylaxe und Behandlung von Niereninsuffizienz geeignet. Die zu verwendenden Dosen können individuell verschieden sein und variieren naturgemäß je nach Art des zu behandelnden Zustandes und der Applikationsform. Im allgemeinen eignen sich jedoch für orale Applikationen an größeren Säugetieren, insbesondere Menschen, Arzneiformen mit einem Wirkstoffgehalt von 0,05 bis 0,2 mg pro Einzeldosis.

Das folgende Beispiel soll die Herstellung einer zur Behandlung von Niereninsuffizienz geeigneten Moxonidin enthaltenden pharmazeutischen Zubereitung näher erläutern, ohne jedoch den Umfang der Anmeldung zu begrenzen.

### Beispiel 1:

### Moxonidin-haltige filmüberzogene Tabletten

| **Zusammensetzung:** | |
|---|---|
| Tablettenkerne: | |
| Moxonidin | 0,010 Teile |
| Lactose | 9,590 Teile |
| Povidone USP | 0,070 Teile |
| Crospovidone USP | 0,300 Teile |
| Magnesiumstearat | 0,030 Teile |
| (Wasser | 0,750 Teile) |
| Gesamtfeststoff | 10,000 Teile |
| | |

| Filmüberzug: | |
|---|---|
| Hydroxypropylmethylcellulose | 0,156 Teile |
| 30 %-ige wäßrige Ethylcellulose-Dispersion | 0,480 Teile |
| (Δ Feststoff) | (0,144) Teile |
| Polyethylenglycol 6000 | 0,030 Teile |
| Titandioxid | 0,150 Teile |
| Talc | 0,1197 Teile |
| Rotes Eisenoxid | 0,0003 Teile |
| (Wasser | 3,864 Teile) |
| Gesamtfeststoff | 0,600 Teile |
| Gesamtmenge an Filmüberzugssuspension | 4,800 Teile |

Zum Überziehen von 10.000 Tablettenkerne zu je 100 mg Gewicht werden 4,8 kg der vorstehenden Filmüberzugssuspension eingesetzt.

### Tablettenkernherstellung:

Das Moxonidin und die Lactose wurden gemischt. Die Mischung wurde mit einer Lösung des Bindemittels Povidone in Wasser durchfeuchtet, gut durchgeknetet und das erhaltene Produkt wurde auf Horden ausgebreitet und bei einer Temperatur von ca. 50 °C bis zu einem Feuchtigkeitsgehalt von höchstens 0,5 % getrocknet. Das getrocknete Produkt wurde durch ein 0,75 mm-Sieb (Frewitt-Maschine) passiert. Nach dem Vermischen des erhaltenen Granulates mit Crospovidone und Magnesiumstearat wurden daraus Tablettenkerne mit einem Gewicht von 100 mg gepreßt, so daß jeder Tablettenkern 0,1 mg Wirkstoff enthielt.

### Herstellung der Filmüberzugssuspension:

Die Hydroxypropylmethylcellulose und das Polyethylenglycol 6000 wurden in einem Teil des Wassers gelöst. Zu dieser Lösung wurde eine Suspension von Talc, Titandioxid und Eisenoxid in dem übrigen Wasser unter Rühren zugefügt. Die erhaltene Suspension wurde unter leichtem Rühren mit der 30 %igen wäßrigen Ethylcellulose-Dispersion verdünnt.

### Filmüberziehen der Tablettenkerne:

Die Filmüberzugssuspension wurde auf die Tablettenkerne in einer Befilmungsapparatur gesprüht, während warme Luft von ca. 70 °C die Tablettenkerne auf eine Temperatur von ca. 45 °C erwärmte. Anschließend wurden die filmüberzogenen Tabletten 16 Stunden bei einer Temperatur von ca. 45 °C getrocknet.

## Patentansprüche

1. Verwendung einer subantihypertensiven Menge der Verbindung 4-Chlor-5-[(4,5-dihydro-1H-imidazol-2-yl)-amino]-6-methoxy-2-methylpyrimidin der Formel I 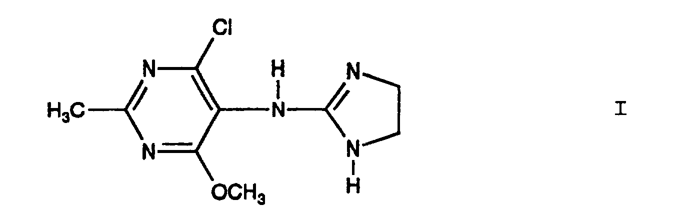 oder dessen physiologisch verträglichen Säureadditionssalzen zur Herstellung von pharmazeutischen Zubereitungen zur Prophylaxe und/oder zur Behandlung von Nierenschädigungen und/oder Niereninsuffizienz in normotensiven Patienten.

2. Verwendung nach Anspruch 1, d.g., daß die normotensiven Patienten spontan normotensiv oder durch ein anderes bludrucksenkendes Mittel als die Verbindung der Formel I oder dessen physiologisch verträglichen Säureadditionssalzen normotensiv gestellt sind.

3. Verwendung nach Anspruch 1, d.g., daß die Verbindung der Formel I oder dessen physiologisch verträglichen Säureadditionssalzen zur Herstellung von oralen pharmazeutischen Zubereitungen, vorzugsweise in einer subantihypertensiven Menge von 0,05 bis 0,2 mg pro Einzeldosis verwendet wird.

4. Verfahren zur Herstellung von pharmazeutischen Zubereitungen zur Prophylaxe und/oder Behandlung von Nierenschädigungen und/oder Niereninsuffizienz in normotensiven Patienten, **dadurch gekennzeichnet, daß** man eine subantihypertensive nephroprotektiv wirksame Menge der Verbindung der Formel I gemäß Anspruch 1, oder deren physiologisch verträglichen Säureadditionssalzen zusammen mit üblichen pharmazeutischen Hilfsstoffen in eine geeignete Arzneiform überführt.

## Claims

1. The use of a sub-antihypertensive quantity of the compound 4-chloro-5-[(4,5-dihydro-1H-imidazol-2-yl)-amino]-6-methoxy-2-methylpyrimidine of Formula I 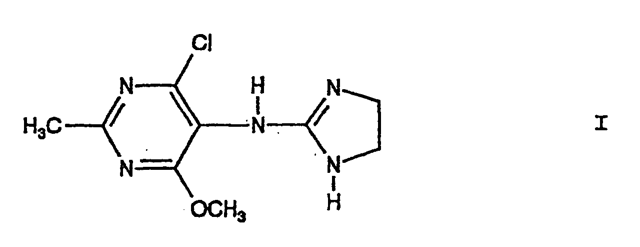 or the physiologically compatible acid addition salts thereof for the preparation of pharmaceutical preparations for the prophylaxis and/or treatment of kidney damage and/or of renal failure in normotensive patients.

2. The use according to Claim 1, **characterised in that** the normotensive patients are spontaneously normotensive or are rendered normotensive by a hypotensive agent other than the compound of Formula I or its physiologically compatible acidaddition salts.

3. The use according to Claim 1, **characterised in that** the compound of Formula I or the physiological [sic] acid addition salts thereof [are used] for the preparation of oral pharmaceutical preparations, preferably in a subantihypertensive quantity of 0.05 to 0.2 mg per individual dose.

4. A method for the preparation of pharmaceutical preparations for the prophylaxis and/or treatment of kidney damage and/or renal failure in normotensive patients, **characterised in that** a sub-antihypertensive nephroprotective quantity of the compound of Formula I according to Claim 1 or the physiologically compatible acid addition salts thereof together with conventional pharmaceutical auxiliaries is converted into a suitable medicament form.

## Revendications

1. Utilisation d'une quantité sub-antihypertensive du composé 4-chloro-5-[(4,5-dihydro-1H-imidazol-2-yl)-amino]-6-méthoxy-2-méthyl-pyrimidine correspondant à la formule I 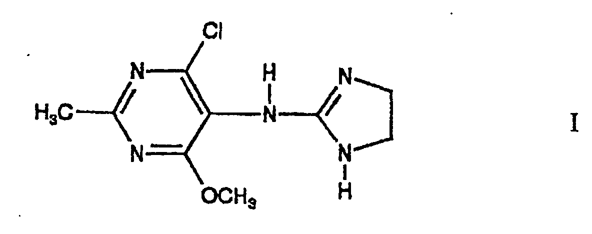 ou de ses sels d'addition acides physiologiquement compatibles pour fabriquer des préparations pharmaceutiques à des fins de prophylaxie et/ou de traitement de lésions rénales et/ou d'insuffisance rénale chez des patients normotendus.

2. Utilisation selon la revendication 1, **caractérisée en ce que** les patients normotendus sont spontanément normotendus ou réglés pour être normotendus par un agent de réduction de la tension autre que le composé de la formule I ou ses sels d'addition acides physiologiquement compatibles.

3. Utilisation selon la revendication 1, **caractérisée en ce que** le composé de formule I ou ses sels d'addition acides physiologiquement compatibles sont utilisés pour fabriquer des préparations pharmaceutiques orales, de préférence dans une quantité sub-antihypertensive comprise entre 0,5 et 0,2 mg par dose simple.

4. Procédé de fabrication de préparations pharmaceutiques destinées à la prophylaxie et/ou au traitement de lésions rénales et/ou d'insuffisance rénale chez des patients normotendus, **caractérisé en ce que** l'on transfère une quantité efficace sub-antihypertensive néphroprotectrice du composé de formule I selon la revendication 1, ou de ses sels d'addition acides physiologiquement compatibles, dans une forme de médicament appropriée, avec des additifs pharmaceutiques usuels.
